# EUROPÄISCHE PATENTSCHRIFT

(11) **EP 1 902 704 B1**
(45) Veröffentlichungstag und Bekanntmachung des Hinweises auf die Patenterteilung: **06.11.2013**
(21) Anmeldenummer: 07016800.0
(22) Anmeldetag: 28.08.2007
(51) Int. Cl.: A61K 8/02, A61K 8/06, A61K 8/81, A61K 8/891, A61K 8/895, A61Q 19/08

(54) **Kosmetische Zusammensetzungen mit einer Siliconelastomer- Kombination**
Cosmetic compositions containing a combination of silicon-elastomers
Compositions cosmétiques comprenantes une combinaison d'élastomères de silicone

(30) Priorität: 31.08.2006 DE 102006040903
(43) Veröffentlichungstag der Anmeldung: 26.03.2008
(73) Patentinhaber: Henkel AG & Co. KGaA, 40589 Düsseldorf (DE)
(72) Erfinder: Frisoli, Christian, 41564 Kaarst (DE); Struwe, Alexandra, 47877 Willich (DE); Waldmann-Laue, Marianne, 40789 Monheim (DE)

(56) Entgegenhaltungen:
- EP-A- 1 064 930
- EP-A- 1 360 955
- EP-A- 1 550 434
- EP-A1- 1 493 421
- WO-A-02/47639
- JP-A- 2005 314 327

## Beschreibung

Moderne kosmetische Zusammensetzungen zur Pflege der Haut, insbesondere der Gesichtshaut, müssen vielfachen Anforderungen des Verbrauchers genügen.

Neben der Erfüllung ihrer eigentlichen Bestimmung - Feuchtigkeitsbindung, Stärkung der Barrierefunktion der Haut und Verbesserung des Erscheinungsbildes der Haut bei Hautcremes, -gelen, -lotionen, -masken usw. - werden weitere kosmetische Effekte erwartet. Bei einer Hautcreme oder einer ähnlichen Zusammensetzung kann das die Faltenbehandlung, Hautaufhellung oder -bräunung, das Kaschieren von Hautunreinheiten und -unebenheiten sein.

Ein weiteres wichtiges Kriterium bei der Beurteilung einer kosmetischen Zusammensetzung durch den Anwender ist der sensorische, insbesondere der taktil-sensorische Eindruck, der bei der Applikation der Zusammensetzung auf die Haut oder die Haare hervorgerufen wird.

Als besonders angenehm wird von vielen Verbrauchern der sensorische Eindruck, den Silicon-Verbindungen, z. B. Siliconöle oder Siliconelastomere erzeugen, empfunden. Siliconöle werden aufgrund ihres Spreit- und Einzugsverhaltens als samtweich und nicht-fettend empfunden; außerdem wirken sie, anders als viele natürliche Fette und Öle, nicht comedogen. Zahlreiche Siliconöle hinterlassen die Haut ohne Fettglanz.

Bei höheren Anteilen an Siliconölen in einer kosmetischen Zusammensetzung können Schwierigkeiten bei der Einstellung der für das jeweilige Anwendungsgebiet geeigneten Viskosität auftreten, häufig in Verbindung mit Problemen bei der Lager- und/oder Temperaturstabilität.

Außerdem wird ein zu hoher Anteil an Siliconölen in einer kosmetischen Zusammensetzung von vielen Verbrauchern nach einer gewissen Zeit als zu wenig hautpflegend, mitunter sogar als austrocknend, empfunden. Es hat sich hier als günstig erweisen, Siliconöl-Wasser-Emulsionen zu formulieren. Die Emulgierung der Siliconöle zusammen mit Wasser hat weitere Vorteile: zahlreiche Wirkstoffe gegen die Hautalterung oder andere negative Hautzustände sind eher wasserlöslich als öllöslich und daher besser oder überhaupt nur mit einer wasserhaltigen Zusammensetzung kompatibel. Nicht zuletzt stellt Wasser einen ökonomisch günstigen Rohstoff dar. Durch den Zusatz von Wasser wird allerdings der taktil-sensorische Eindruck, der direkt bei der Applikation der Zusammensetzung auf die Haut hervorgerufen wird, negativ beeinträchtigt. Außerdem sieht sich der Fachmann bei der Herstellung von Emulsionen, thermodynamisch instabilen Systemen, immer mit einem Stabilitätsproblem konfrontiert, für das er eine optimale Lösung finden muss.

### Stand der Technik

EP 1097703 A1 offenbart eine kosmetische Zusammensetzung zur Gesichtspflege, die als Öl-in-Wasser-Emulsion formuliert ist und eine wässrige Siliconelastomer-Dispersion sowie einen Verdicker auf Basis eines Polymerlatex enthält.

EP 1550432 A1 offenbart einen wasserhaltigen Gesichtspuder, der eine wässrige Siliconelastomer-Dispersion sowie weitere partikuläre Bestandteile enthält, wobei das Gewichtsverhältnis von Siliconelastomer und weiterem partikulären Bestandteil mehr als 1,2 und maximal 2,5 beträgt.

EP 1097695 A1 offenbart eine kosmetische Zusammensetzung zur Gesichtspflege, die als Wasser-in-Öl-Emulsion formuliert ist und eine wässrige Siliconelastomer-Dispersion sowie ein Silicon-basiertes Siliconelastomergel enthält.

EP 1064930 und EP 1360955 A2 offenbaren kosmetische Zusammensetzungen zur Gesichtspflege, die eine wässrige Siliconelastomer-Dispersion sowie ein Silicon-basiertes Siliconelastomergel enthalten.

EP 1493421 A1 offenbart kosmetische Zusammensetzungen zur optischen Egalisierung des Hautreliefs, die einen primären C₂-C₃-Alkohol, ein Silicon-basiertes Siliconelästomergel und ein im Wesentlichen lineares Silicon, das in Form einer wässrigen Dispersion eingesetzt wird, enthaften.

### Aufgabenstellung

Eine Aufgabe der vorliegenden Erfindung war die Bereitstellung einer ästhetisch ansprechenden kosmetischen Zusammensetzung mit hervorragenden pflegenden und konditionierenden Eigenschaften. Eine weitere Aufgabe der vorliegenden Erfindung war die Bereitstellung einer sensorisch, insbesondere taktil-sensorisch, ansprechenden kosmetischen Zusammensetzung. Eine weitere Aufgabe der vorliegenden Efindung war die Bereitstellung einer lager- und/oder temperaturstabilen kosmetischen Zusammensetzung auf Basis einer wasser- und ölhaltigen Zusammensetzung.

Überraschend wurde nun gefunden, dass durch die Kombination gemäß Anspruch 1 aus mindestens einer wässrigen Phase, in der ein Siliconelastomer a vorliegt, und mindestens einer Ölphase, in der mindestens ein Siliconelastomer b in einem Silicon-basierten Gel vorliegt, wobei das Gewichtsverhältnis von Siliconelastomer a zu Siliconelastomer b größer als 1 ist, besonders lager- und temperaturstabile kosmetische Zusammensetzung mit einem außergewöhnlich angenehmen sensorischen, insbesondere taktil-sensorischen, Eindruck hergestellt werden können.

Ein erster Gegenstand der vorliegenden Erfindung ist eine kosmetische Zusammensetzung, die in einem geeigneten kosmetischen Träger folgende Bestandteile enthält:
- mindestens ein Siliconelastomer a, das in Form einer wässrigen Dispersion eingesetzt wird oder in der wässrigen Phase dispergiert ist,
- mindestens ein Siliconelastomer b, das in einem Silicon-basierten Gel vorliegt,
   - wobei das Gewichtsverhältnis von Siliconelastomer a zu Siliconelastomer b größer als 1 ist,
- mindestens eine wässrige Phase,
- mindestens eine Ölphase,
- mindestens einen partikelförmigen Wirkstoff, ausgewählt aus organischen kosmetischen Adsorbentien mit mittleren Partikeldurchmessern von 5 - 30 µm, bevorzugt 9 - 25 µm, in einer Gesamtmenge von 3 - 10 Gew.%, bezogen auf das Gesamtgewicht der Zusammensetzung, enthalten ist, wobei der partikelförmige Wirkstoff zu mehr als 50 Gew.% sphärische Partikel enthält.

Eine bevorzugte Ausführungsform der Erfindung ist dadurch gekennzeichnet, dass das Gewichtsverhältnis von Siliconelastomer a zu Siliconelastomer b im Bereich von 1,5 - 50, besonders bevorzugt 2 - 30 und außerordentlich bevorzugt 5 - 25, liegt. Überraschend wurde festgestellt, dass sich mit diesen bevorzugten Gewichtsverhältnissen Zusammensetzungen mit besonders hoher Lagerstabilität herstellen lassen.

Eine weitere bevorzugte Ausführungsform der Erfindung ist dadurch gekennzeichnet, dass mindestens eines der Siliconelastomere a) oder b) erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist.

Erfindungsgemäß besonders bevorzugte Organopolysiloxane mit mindestens 2 C₂ - C₁₀ - AlkenylGruppen mit terminaler Doppelbindung im Molekül sind ausgewählt aus Methylvinylsiloxanen, Methyl-vinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethyl-siloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethyl-siloxy-Endgruppen und cyclischen Dimethylsiloxan-Methylhydrogen-siloxan-Copolymeren.

Entsprechende erfindungsgemäß bevorzugte Siliconelastomere sind kommerziell erhältlich, beispielsweise unter den Handelsnamen SFE 167, einem Cetearyl DimethiconeNinyl Dimethicone Crosspolymer von GE Silicones (Waterford, N.Y.), Dow Coming 9506 Cosmetic Powder und Dow Coming 9509 Cosmetic Powder, jeweils von Dow Corning. Diese Siliconelastomere lassen sich als erfindungsgemäß bevorzugte Siliconelastomere a in einer wässrigen Phase dispergieren.

Erfindungsgemäß besonders bevorzugte Siliconelastomere a, die bereits als Rohstoff in einer wässrigen Phase dispergiert sind, sind ebenfalls kommerziell erhältlich, beispielsweise unter den Handelsnamen BY 29-119 und BY 29-122, jeweils von Dow Corning. Diese Handelsprodukte weisen einen Siliconelastomergehalt von 58- 68 Gew.%, also durchschnittlich 63 Gew.%, auf. Weiterhin bevorzugt sind die Produkte Gransil^{®} LTX (INCI-Bezeichnung : Cyclopentasiloxane (and) Polysilicone-11 (and) Water (and) Laureth-4), Gransil^{®} LTX-10- (INCI-Bezeichnung : Isododecane (and) Polysilicone-11 (and) Water (and) Laureth-4) und Gransil^{®} SQC (INCI-Bezeichnung : Cyclopentasiloxane (and) Dimethicone/ Divinyldimethicone/Silsesquioxane Crosspolymer (and) Water (and) Laureth-12), jeweils von Grant Industries.

Erfindungsgemäß besonders bevorzugte Siliconelastomere b, die als Rohstoff bereits in einem bei Raumtemperatur unter Normalbedingungen flüssigen Silicon vorgequollen vorliegen und ein Silicon-basiertes Gel darstellen, sind ebenfalls kommerziell erhältlich, beispielsweise unter den Handelsnamen SFE 168, ein Cyclomethicone (and) DimethiconeNinyl Dimethicone Crosspolymer von GE Silicones, Vinyl Dimethicone Crosspolymere, enthalten in KSG-15 (Cyclomethicone (and) DimethiconeNinyl Dimethicone Crosspolymer, Siliconelastomergehalt 4 - 10 Gew.-%), KSG-16 (Dimethicone (and) DimethiconeNinyl Dimethicone Crosspolymer, Siliconelastomergehalt 20 - 30 Gew.-%), KSG-17 (Cyclomethicone (and) DimethiconeNinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil^{®}-Serie, insbesondere Gransil SR-CYC (Cyclomethi-cone and Stearyl-VinyUhydromethylsiloxane Copolymer), Gransil^{®} RPS Gel (INCI-Bezeichnung: Cyclopentasiloxane and Polysilicone-11), Gransil^{®}GCM-4 (INCI-Bezeichnung: Cyclotetrasiloxane and Polysilicone-11), Gransil^{®}GCM-5 (INCI-Bezeichnung: Cyclopentasiloxane and Polysilicone-11), Gransil^{®} RPS (INCI-Bezeichnung: Cyclopentasiloxane and Polysilicone-11), GI-CD 10 (INCI-Bezeichnung: Cyclopentasiloxane (and) Stearoxymethicone/Dimethicone Copolymer (and) Dimethicone), Gransil^{®} IDS (INCI-Bezeichnung: Isododecane (and) Cyclotetrasiloxane (and) Polysilicone-11), Gransil^{®}PC-12 (INCI-Bezeichnung: Isododecane (and) Polysilicone-11), Gransil^{®}IDS-5 (INCl-Bezeichnung: Isododecane (and) Cyclopentasiloxane (and) Polysilicone-11), Gransil^{®}APK-1 (INCI-Bezeichnung: Dimethicone and Cyclopentasiloxane and Polysilicone-11 and Nylon-12 and Methyl Methacrylate/Acrylonitrile Copolymer and PEG-10 Dimethicone and Polysorbate-40 and Isohexadecane and Ammonium Polyacryloyldimethyl Taurate), Gransif^{®}DMCM-5 (INCI-Bezeichnung: Dimethicone and Cyclopentasiloxane and Polysilicone-11), Gransil^{®}DMG-6 mit Dimethicone (6 cSt) (INCI-Bezeichnung: Dimethicone and Polysilicone-11), Gransil^{®}DMG-20 mit Dimethicone (20 cSt) (INCI-Bezeichnung: Dimethicone and Polysilicone-11), Gransil^{®} AM-8 Gel (INCI-Bezeichnung: Caprylyl Methicone and Cyclopentasiloxane and Polysilicone-11), Gransil^{®} DM 5 mit Dimethicone (5 cSt) (INCI-Bezeichnung: Dimethicone and Polysilicone-11), Gransil^{®} DMID (INCI-Bezeichnung: Dimethicone and Isododecane and Polysilicone-11), Gransil^{®} PM (INCI-Bezeichnung: Phenyl Trimethicone and Polysilicone-11), Gransil^{®} ININ (INCI-Bezeichnung: Isononyl Isononanoate (and) Polysilicone-11).

Ebenfalls mit Vorzug in den erfindungsgemäßen Zusammensetzungen einsetzbar sind Siliconelastomere b, die als Rohstoff bereits in einem bei Raumtemperatur unter Normalbedingungen flüssigen Silicon, gemischt mit einem Nicht-Silicon-haltigen Öl, Fett oder Wachs, vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil^{®} MLB (INCI-Bezeichnung: Cyclopentasiloxane and Polysilicone-11 and Beeswax), Gransil^{®} PS (INCI-Bezeichnung: Cyclotetrasiloxane and Polysilicone-11 and Petrolatum), Gransil^{®} PS-5 (INCI-Bezeichnung: Cyclopentasiloxane and Polysilicone-11 and Petrolatum), Gransil^{®}DMG-20 P mit Dimethicone (20 cSt) und Petrolatum (INCI-Bezeichnung: Dimethicone and Polysilicone-11 and Petrolatum), Gransil^{®} RJO (INCI-Bezeichnung: Cyclopentasiloxane and Polysilicone-11 and Jojoba Oil), Gransil^{®} LANO (INCI-Bezeichnung: Cyclopentasiloxane and Polysilicone-11 and Lanolin), Gransil^{®} OHS-5 (INCI-Bezeichnung: Cyclopentasiloxane and Polysilicone-11 and Octyl Hydroxystearate) und Gransil^{®} DML (INCI-Bezeichnung: Dimethicone (and) Neopentyl Glycol Diheptanoate (and) Polysilicone-11).

Eine weitere bevorzugte Ausführungsform der Erfindung ist dadurch gekennzeichnet, dass mindestens eines der Siliconelastomere a) oder b) durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist.

Erfindungsgemäß besonders bevorzugte vernetzende alpha, omega-Diene weisen die Formel CH₂=CH(CH₂)ₓCH=CH₂ mit x = 1 - 20 auf. Besonders bevorzugte alpha, omega-Diene sind ausgewählt aus 1,4-Pentadien, 1,5-Hexadien, 1,6- Heptadien, 1,7-Octadien, 1,8-Nonadien, 1,11-Dodecadien, 1,13-Tetradecadien und 1,19- Eicosadien.

Eine weitere bevorzugte Ausführungsform der Erfindung ist dadurch gekennzeichnet, dass mindestens eines der Siliconelastomere a) oder b) emulgierende Eigenschaften aufweist. Es wurde überraschend festgestellt, dass die dieser Anmeldung zugrunde liegenden Aufgaben dadurch besonders gut gelöst wurden; insbesondere die Temperaturstabilität der erfindungsgemäßen Zusammensetzungen konnte verbessert werden. Erfindungsgemäß besonders bevorzugt ist es, wenn das Siliconelastomer b) emulgierende Eigenschaften aufweist. Die emulgierenden Eigenschaften sind bevorzugt erhältlich mit Siliconelastomeren aus vernetzten Organopolysiloxanen, die am Polysiloxan-Gerüst Polyoxyethylen- und/oder Polyoxypropylen-Gruppen als funktionelle Gruppen enthalten. Diese Gruppen können endständig und/ oder als Seitengruppen zur Polysiloxan-Kette angeordnet sein.

Erfindungsgemäß können sowohl nicht-emulgierende als auch emulgierende Siliconelastomere sowie deren Mischungen eingesetzt werden.

Erfindungsgemäß besonders bevorzugte nicht-emulgierende Siliconelastomere b, die mit mindestens einem alpha, omega-Dien vernetzte Organopolysiloxane in einem Silicon-basierten Gel enthalten, sind im Handel erhältlich, beispielsweise von Dow Corning die Produkte DC 9040 (INCI-Bezeichnung: Cyclomethicone (and) Dimethicone Crosspolymer, Siliconelastomergehalt 12 - 14 Gew.-%) und DC 9041 (INCI-Bezeichnung: Dimethicone (and) Dimethicone Crosspolymer).

Erfindungsgemäß besonders bevorzugte emulgierende Siliconelastomere b, die mit mindestens einem alpha, omega-Dien vernetzte Ethylenoxid-substituierte Organopolysiloxane in einem Silicon-basierten Gel enthalten, sind im Handel erhältlich, beispielsweise von Dow Corning die Produkte DC 9010 und DC 9011 (INCI-Bezeichnung: Cyclopentasiloxane (and) PEG-12 Dimethicone Crosspolymer).

Erfindungsgemäß besonders bevorzugte emulgierende Siliconelastomere b, die vernetzte Ethylenoxid-substituierte Organopolysiloxane in einem Silicon-basierten Gel enthalten, sind im Handel erhältlich, beispielsweise die Produkte KSG-21, KSG-31, KSG-31X und KSG-32 von Shin-Etsu.

Eine weitere bevorzugte Ausführungsform der Erfindung ist dadurch gekennzeichnet, dass das mindestens eine Siliconelastomer a, das in der wässrigen Phase dispergiert vorliegt, in einer Gesamtmenge von 0,5 - 30 Gew.%, bevorzugt 2,0 - 20 Gew.% und besonders bevorzugt 10 - 15 Gew.%, jeweils bezogen auf die gesamte kosmetische Zusammensetzung, enthalten ist.

Eine weitere bevorzugte Ausführungsform der Erfindung ist dadurch gekennzeichnet, dass das mindestens eine Siliconelastomer b, das in einem Silicon-basierten Gel vorliegt, in einer Gesamtmenge von 0,05 - 5 Gew.%, bevorzugt 0,2 - 2 Gew.% und besonders bevorzugt 0,5 - 1 Gew.%, jeweils bezogen auf die gesamte kosmetische Zusammensetzung, enthalten ist.

Eine weitere bevorzugte Ausführungsform der Erfindung ist dadurch gekennzeichnet, dass die Zusammensetzung als Öl-in-Wasser-Emulsion vorliegt. Diese Systeme sind im Hinblick auf die Lagerstabilität und/oder die sensorischen Eigenschaften den Wasser-in-Öl-Emulsionen häufig überlegen.

Weiterhin hat sich überraschend gezeigt, dass die Stabilitätseigenschaften und/oder die sensorischen Eigenschaften der erfindungsgemäßen Zusammensetzungen weiter verbessert werden konnten durch den Zusatz mindestens eines polymeren Verdickers in Form eines verzweigten oder vernetzten Polyelektrolyten, wobei der Polyelektrolyt ausgewählt ist aus einem Copolymer aus mindestens zwei Monomer-Typen, wobei mindestens ein Monomer eine starke Säurefunktion enthält, die teilweise oder vollständig neutralisiert ist, und mindestens ein Monomer entweder neutral ist oder eine schwache Säurefunktion enthält. Eine weitere bevorzugte Ausführungsform der Erfindung ist dadurch gekennzeichnet, dass mindestens ein polymerer Verdicker in Form eines verzweigten oder vernetzten Polyelektrolyten enthalten ist, wobei der Polyelektrolyt ausgewählt ist aus einem Copolymer aus mindestens zwei Monomer-Typen, wobei mindestens ein Monomer eine starke Säurefunktion enthält, die teilweise oder vollständig neutralisiert ist, und mindestens ein Monomer entweder neutral ist oder eine schwache Säurefunktion enthält.

In einer bevorzugten Ausführungsform der Erfindung sind die Polyelektrolytmonomeren, die eine schwache Säurefunktion enthalten, ausgewählt aus den Monomeren, die eine Carboxylgruppe -COOH enthalten, die teilweise oder vollständig neutralisiert ist. Besonders bevorzugte Beispiele für solche schwach sauren Polyelektrolytmonomeren sind Acrylsäure, Methacrylsäure, Itaconsäure und Maleinsäure.

In einer weiteren bevorzugten Ausführungsform der vorliegenden Erfindung sind die Polyelektrolytmonomere mit einer starken Säuregruppe ausgewählt aus Monomeren, die mit einer Sulfonsäuregruppe -SO₃H oder einer Phosphonsäuregruppe funktionalisiert sind. Ein besonders bevorzugtes Polyelektrolytmonomer mit starker Säurefunktion ist 2-Methyl-2[(1-oxo-2-propenyl)amino]-1-propan-sulfonsäure, die teilweise oder vollständig neutralisiert ist.

Eine weitere bevorzugte Ausführungsform der Erfindung ist dadurch gekennzeichnet, dass die Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Salz vorliegen. Besonders bevorzugt sind die Natriumsalze und die Ammoniumsalze.

Eine weitere bevorzugte Ausführungsform der Erfindung ist dadurch gekennzeichnet, dass das neutrale Polyelektrolytmonomer ausgewählt ist aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der vorstehend genannten Ester mit einem Molekulargewicht zwischen 400 und 1000 g/mol, Acrylamid sowie Vinylpyrrolidon.

Erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Vinylpyrrolidon. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Vinylpyrrolidon, die in Form des Ammoniumsalzes kommerziell erhältlich sind, z. B. unter dem Handelsnamen Aristoflex^{®} AVC von der Firma Clariant.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Hydroxyethylacrylat. Erfindungsgemäß außerordentlich bevorzugt sind teilweise oder vollständig neutralisierte, vernetzte Copolymere aus 2-Methyl-2[(1-oxo-2-propenyl)amino]-1-propansulfon-säure (AMPS) und Hydroxyethylacrylat, die in Form eines inversen Latex kommerziell erhältlich sind, z. B. unter dem Handelsnamen Simulgel^{®}NS von der Firma Seppic.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Acrylsäure, wobei die Säuregruppen teilweise oder vollständig neutralisiert sein können. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Acrylsäure, die in Form eines inversen Latex kommerziell erhältlich sind, z. B. unter den Handelsnamen Simulgel^{®} EG, Sepiplus^{®} 400 und Simulgel^{®} EPG von der Firma Seppic.

Weitere erfindungsgemäß besonders bevorzugte verdickende Polymere sind teilweise oder vollständig neutralisierte, vernetzte Copolymere aus 2-Methyl-2[(1-oxo-2-propenyl)amino]-1-propansulfonsäure (AMPS) und Acrylamid. Erfindungsgemäß außerordentlich bevorzugt sind teilweise oder vollständig neutralisierte, vernetzte Copolymere aus 2-Methyl-2[(1-oxo-2-propenyl)amino]-1-propansulfonsäure (AMPS) und Acrylamid, die in Form eines inversen Latex kommerziell erhältlich sind, z. B. unter den Handelsnamen Simulgel^{®} 600, Sepiplus^{®} 400 und Sepigel^{®} 305 von der Firma Seppic.

Sofern es sich bei den erfindungsgemäß besonders bevorzugten verdickenden Polymeren um inverse Polymerlatices handelt, ist diesen gemein, dass sie mindestens ein Öl sowie mindestens einen Öl-in-Wasser-Emulgator enthalten. Besonders bevorzugte Öle sind ausgewählt aus weißen Mineralölen, Squalan, Polyisobuten und hydriertem Polyisobuten. Besonders bevorzugte Öl-in-Wasser-Emulgatoren sind ausgewählt aus den Ethylenoxid-Addukten von Verbindungen wie bevorzugt Sorbitanoleat, Ricinusöl, das gewünschtenfalls gehärtet ist, Sorbitanlaurat und Laurylalkohol, wobei der Ethoxylierungsgrad so hoch ist, dass die Emulgatoren als Öl-in-Wasser-Emulgator wirken. Weitere besonders bevorzugte Öl-in-Wasser-Emulgatoren sind ausgewählt aus Alkyl-(oligo)-glucosiden, insbesondere Alkyl-(oligo)-glucosiden mit einem linearen C₆-, C₈-, C₁₀- und C₁₂-Alkylrest sowie Mischungen hiervon, insbesondere C₈/C₁₀-Alkyloligoglucoside, besonders bevorzugt C₈/C₁₀-Alkyloligoglucoside, jeweils mit einem Oligomerisierungsgrad von 1,1 - 1,4.

Sofern es sich bei den erfindungsgemäß besonders bevorzugten verdickenden Polymeren um inverse Polymerlatices handelt, ist diesen gemein, dass sie bevorzugt einen Polymergehalt von 30- 90 Gew.-%, besonders bevorzugt 50 - 80 Gew.% und außerordentlich bevorzugt 60 - 75 Gew.%, jeweils bezogen auf den gesamten Latex, aufweisen.

Bezogen auf die gesamte erfindungsgemäße kosmetische Zusammensetzung, ist das verdickende Copolymer mit mindestens einem Monomer mit starker Säurefunktion und mindestens einem neutralen Monomer oder Monomer mit schwacher Säurefunktion bevorzugt in Gesamtmengen von 0,1 - 5 Gew.-%, besonders bevorzugt 0,3 - 3 Gew.% und außerordentlich bevorzugt 0,5 - 2,5 Gew.-%, jeweils bezogen auf die gesamte erfindungsgemäße kosmetische Zusammensetzung, enthalten.

Weiterhin hat sich überraschend gezeigt, dass die erfindungsgemäßen Zusammensetzungen durch ihre Stabilitätseigenschaften und/oder sensorischen Eigenschaften hervorragend als Vehikel oder Cremegrundlage für Abdeck- und Schminkzusammensetzungen geeignet sind, die durch einen Gehalt an partikelförmigen Adsorbentien und/oder Pigmenten gekennzeichnet sind. Die Erfindung ist daher dadurch gekennzeichnet, dass in den erfindungsgemäßen Zusammensetzungen mindestens ein partikelförmiger Wirkstoff, ausgewählt aus organischen kosmetischen Adsorbentien mit mittleren Partikeldurchmessern von 5 - 30 µm und bevorzugt 9-25 µm, der zu mehr als 50 Gew.% sphärische Partikel enthält, in einer Gesamtmenge von 3 - 10 Gew.% und bevorzugt 3 - 5 Gew.%, jeweils bezogen auf das Gesamtgewicht der Zusammensetzung, enthalten ist.

Die Bestimmung des mittleren Partikeldurchmessers erfolgt durch eine Partikelgrößenanalyse mittels Siebanalyse nach DIN 66165-1: 1987-04 und DIN 66165-2: 1987-04.

Bei nicht-sphärischen Partikeln bezieht sich die Angabe des mittleren Partikeldurchmessers auf die größte Längsausdehnung.

Eine weitere bevorzugte Ausführungsform der Erfindung ist dadurch gekennzeichnet, dass der partikelförmige Wirkstoff ausgewählt ist aus folgenden organischen Adsorbentien: Stärken und Stärkederivaten, die chemisch und/oder physikalisch modifiziert sein können, Cellulosepulvern, Lactoglobulinderivaten, insbesondere Natrium-C₈₋₁₆-Isoalkylsuccinyllactoglobulinsulfonat, Polymerpulvern aus Polyolefinen, insbesondere Polyethylen-Pulvern und Polypropylen-Pulvern, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, sowie Mischungen hiervon.

Besonders bevorzugte organische Adsorbentien sind ausgewählt aus Stärken und Stärkederivaten, die chemisch und/oder physikalisch modifiziert sein können, insbesondere modifizierten Stärkederivaten vom Typ der Stärkeoctenylsuccinatester, insbesondere der Aluminiumstärkeoctenylsuccinatester, beispielsweise erhältlich unter der Bezeichung DRY FLO^{®} von National Starch, Cellulosepulvern, Lactoglobulinderivaten, insbesondere Natrium-C₈₋₁₆-Isoalkylsuccinyllactoglobulinsulfonat, z. B. als Handelsprodukt Biopol^{®} OE von Brooks Industries erhältlich, Polymerpulvern aus Polyolefinen, insbesondere Polyethylen-Pulvern und Polypropylen-Pulvern, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, wobei die Polymerpulver in einer besonders bevorzugten Ausführungsform der Erfindung vernetzt sein können, sowie Mischungen der genannten Substanzen.

Erfindungsgemäß besonders bevorzugte Polymerpulver auf Basis eines Polymethacrylat-Copolymers sind z. B. als Handelsprodukt Polytrap^{®} 6603 (Dow Coming) erhältlich. Andere erfindungsgemäß besonders bevorzugte Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere erfindungsgemäß besonders bevorzugte Polymerpulver sind ausgewählt aus vernetzten Polymethacrylaten (insbesondere den Handelsprodukten Micropearl^{®} M von SEPPIC oder Plastic Powder A von NIKKOL), vernetzten Polymethylmethacrylaten (Micropearl^{®} M 305 und Micropearl^{®} M 310 von SEPPIC sowie Covabead von Cognis, insbesondere Covabead LH 85), Styrol-Divinylbenzol-Copolymeren (z. B. Plastic Powder FP von NIKKOL), Polyethylen- und Polypropylen-Pulvern (z. B. ACCUREL^{®} EP 400 von AKZO) oder auch Siliconpolymeren (z. B. Silicone Powder X2-1605 von Dow Corning).

Besonders bevorzugte partikelförmige Wirkstoffe sind ausgewählt aus vernetzten Polymethylmethacrylaten. Besonders bevorzugte vernetzte Polymethylmethacrylate weisen einen mittleren Partikeldurchmesser von 5 - 30 µm und bevorzugt 9 - 25 µm auf. Außerordentlich bevorzugt ist das Handelsprodukt Covabead LH 85 von Cognis.

Weiterhin hat sich überraschend gezeigt, dass erfindungsgemäße Zusammensetzungen mit einem Gehalt an mindestens einem partikelförmigen Wirkstoff, ausgewählt aus vernetzten Polymethacrylaten und vernetzten Polymethylmethacrylaten, besonders herausragende sensorische und Lagerstabilitätseigenschaften aufweisen. Eine weitere bevorzugte Ausführungsform der Erfindung ist daher dadurch gekennzeichnet, dass der partikelförmige Wirkstoff ausgewählt ist aus vernetzten Polymethacrylaten und vernetzten Polymethylmethacrylaten.

Eine weitere bevorzugte Ausführungsform der Erfindung ist dadurch gekennzeichnet, dass der partikelförmige Wirkstoff Hohlräume aufweist.

Eine weitere bevorzugte Ausführungsform der Erfindung ist dadurch gekennzeichnet, dass der partikelförmige Wirkstoff eine durchschnittliche spezifische Oberfläche im Bereich von 50 - 250 m²/g, bevorzugt 70 - 200 m²/g und besonders bevorzugt 80 -100 m²/g aufweist.

Die spezifische Oberfläche wird nach DIN ISO 9277: 2003-05 bestimmt (BET-Methode), vereinfacht auch nach DIN 66132: 1975-07.

Ein weiterer Gegenstand der vorliegenden Erfindung ist die Verwendung einer kosmetischen Zusammensetzung gemäß einem der Patentansprüche 1 - 20 zur nicht-therapeutischen, kosmetischen Behandlung und/oder Minimierung von Hautfalten und -fältchen und/oder zur Verbesserung des optischen Erscheinungsbildes der Haut.

Ein weiterer Gegenstand der vorliegenden Erfindung ist ein nicht-therapeutisches, kosmetisches Verfahren zur nicht-therapeutischen, kosmetischen Behandlung und/oder Minimierung von Hautfalten und -fältchen und/oder zur Verbesserung des optischen Erscheinungsbildes der Haut, bei dem eine kosmetische Zusammensetzung gemäß einem der Patentansprüche 1 - 20 auf die Haut aufgetragen wird.

Die langzeitstabile Einarbeitung von Lichtschutzfilter-Substanzen in Zusammensetzungen, die eine Öl-phase und eine Wasserphase aufweisen, gestaltet sich häufig als sehr schwierig. Organische Lichtschutzfilter-Substanzen sind in den üblichen kosmetischen Ölen und Lösemitteln wenig löslich oder bauen sich darin schnell ab. Überraschend hat sich gezeigt, dass die erfindungsgemäßen Zusammensetzungen durch ihre Stabilitätseigenschaften und/oder sensorischen Eigenschaften hervorragend als Vehikel oder Cremegrundlage für Lichtschutzzusammensetzungen mit einem Gehalt an Lichtschutzfiltern geeignet sind.

Die folgenden Beispiele sollen die Erfindung verdeutlichen, ohne sie hierauf zu beschränken.

### Beispiel 1) O/W - Hautcreme zur Faltenbehandlung

| Bestandteil | INCI-Bezeichnung | Menge (Gew.-%) |
|---|---|---|
| BY 29-119 | DimethiconeNinyl Dimethicone Crosspolymer, Aqua, C12-14 Pareth-12 | 20 |
| Dow Corning 9040 | Cyclomethicone and Dimethicone Crosspolymer | 4,00 |
| Sojalecithin | | 0,50 |
| Isopropylstearat | | 2,00 |
| Myritol^{®} 318 | Caprylic/Capric Triglyceride | 1,00 |
| Tocopherylacetat | | 0,50 |
| Cutina^{®} MD-V | Glyceryl Stearate | 1,00 |
| Cetearylalkohol | | 1,50 |
| Dimethicone | | 5,00 |
| Propylparaben | | 0,20 |
| Weizenproteinhydrolysat | | 1,00 |
| Glycerin | | 5,00 |
| 1,6-Hexandiol | | 6,00 |
| Methylparaben | | 0,20 |
| Tego Carbomer 2%ig | Carbomer | 15,00 |
| Dimethylsilanol-Hyaluronat | | 0,20 |
| Algenextrakt | | 1,00 |
| 1,2-Propylenglycol | | 5,00 |
| Dimethylmethoxychromanol-6 | | 0,01 |
| Simulgel^{®} NS | Hydroxyethyl Acrylate/Sodium Acryloyldimethyltaurate Copolymer, Squalane, Polysorbate 60 | 2,00 |
| Covabead LH 85 | Methylmethacrylate Crosspolymer | 3,00 |
| NaOH 10%ige Lsg. | | 0,23 |
| Mica | | 3,00 |
| Wasser | | ad 100 |

### Beispiel 3) Make up auf Basis einer Öl-in-Wasser-Emulsion

| | |
|---|---|
| Glycerin 86% | 5,000000 |
| Kronos 1171 | 4,000000 |
| Eisenoxid Gelb 10 E 172 | 1,000000 |
| Eisenoxid Rot 30 E 172 | 0,400000 |
| Eisenoxid Schwarz 80 E 172 | 0,200000 |
| DSH-C N | 3,000000 |
| Acnacidol PG | 1,500000 |
| Dow Corning BY 29-119 | 20,000000 |
| Dow Corning 9040 | 5,000000 |
| Dow Corning 245 Fluid | 12,000000 |
| Parfum | 0,200000 |
| Simulgel NS | 2,000000 |
| Covabead LH 85 | 3,000000 |
| 1,2-Octandiol | 1,000000 |
| Phenoxyethanol, rein | 0,500000 |
| Matrixyl 3000 | 2,000000 |
| Propandiol-1,2 | 2,000000 |
| Methylparaben | 0,200000 |
| Propylparaben | 0,200000 |
| Wasser, vollentsalzt | ad 100,000000 |

| | |
|---|---|
| Acnacidol PG: PROPYLENE GLYCOL, 10-HYDROXYDECANOIC ACID, SEBACIC ACID, 1,10-DECANEDIOL | |

## Patentansprüche

1. Kosmetische Zusammensetzung, enthaltend in einem geeigneten kosmetischen Träger
a) mindestens ein Siliconelastomer, das in Form einer wässrigen Dispersion eingesetzt wird oder in der wässrigen Phase dispergiert ist,
b) mindestens ein Siliconelastomer, das in einem Silicon-basierten Gel vorliegt,
c) mindestens eine wässrige Phase,
d) mindestens eine Ölphase,
wobei das Gewichtsverhältnis von Siliconelastomer a zu Siliconelastomer b größer als 1 ist und wobei mindestens ein partikelförmiger Wirkstoff, ausgewählt aus organischen kosmetischen Adsorbentien mit mittleren Partikeldurchmessern von 5 - 30 µm, bevorzugt 9 - 25 µm, der zu mehr als 50 Gew.% sphärische Partikel enthält und in einer Gesamtmenge von 3 - 10 Gew.-%, bezogen auf das Gesamtgewicht der Zusammensetzung, enthalten ist.

2. Zusammensetzung gemäß Anspruch 1, **dadurch gekennzeichnet, dass** das Gewichtsverhältnis von Siliconelastomer a zu Siliconelastomer b im Bereich von 1,5 - 50, bevorzugt 2 - 30 und besonders bevorzugt 5 - 25, liegt.

3. Zusammensetzung gemäß einem der vorhergehenden Ansprüche, **dadurch gekennzeichnet, dass** mindestens eines der Siliconelastomere a) oder b) erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist.

4. Zusammensetzung gemäß einem der vorhergehenden Ansprüche, **dadurch gekennzeichnet, dass** mindestens eines der Siliconelastomere a) oder b) durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist.

5. Zusammensetzung gemäß einem der vorhergehenden Ansprüche, **dadurch gekennzeichnet, dass** das Siliconelastomer ausgewählt ist aus nicht-emulgierenden und emulgierenden Siliconelastomeren.

6. Zusammensetzung gemäß einem der vorhergehenden Ansprüche, **dadurch gekennzeichnet, dass** das Organopolysiloxan mit mindestens 2 C₂ - C₁₀-Alkenyl-Gruppen mit terminaler Doppelbindung im Molekül ausgewählt ist aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

7. Zusammensetzung gemäß einem der vorhergehenden Ansprüche, **dadurch gekennzeichnet, dass** das vernetzende Organopolysiloxan mit mindestens zwei Silicon-gebundenen Wasserstoffatomen ausgewählt ist aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-Siloxan-Copolymeren.

8. Zusammensetzung gemäß einem der vorhergehenden Ansprüche, **dadurch gekennzeichnet, dass** das mindestens eine Siliconelastomer a, das in der wässrigen Phase dispergiert vorliegt, in einer Gesamtmenge von 0,5 - 30 Gew.%, bevorzugt 2,0 - 20 Gew.% und besonders bevorzugt 10 - 15 Gew.%, jeweils bezogen auf die gesamte kosmetische Zusammensetzung, enthalten ist.

9. Zusammensetzung gemäß einem der vorhergehenden Ansprüche, **dadurch gekennzeichnet, dass** das mindestens eine Siliconelastomer b, das in einem Silicon-basierten Gel vorliegt, in einer Gesamtmenge von 0,05 - 5 Gew.%, bevorzugt 0,2 - 2 Gew.% und besonders bevorzugt 0,5 - 1 Gew.%, jeweils bezogen auf die gesamte kosmetische Zusammensetzung, enthalten ist.

10. Zusammensetzung gemäß einem der vorhergehenden Ansprüche, **dadurch gekennzeichnet, dass** der partikelförmige Wirkstoff ausgewählt ist aus vernetzten Polymethacrylaten und vernetzten Polymethylmethacrylaten.

11. Zusammensetzung gemäß einem der vorhergehenden Ansprüche, **dadurch gekennzeichnet, dass** der partikelförmige Wirkstoff Hohlräume aufweist.

12. Zusammensetzung gemäß einem der vorhergehenden Ansprüche, **dadurch gekennzeichnet, dass** die Zusammensetzung als Öl-in-Wasser-Emulsion vorliegt.

13. Zusammensetzung gemäß einem der vorhergehenden Ansprüche, **dadurch gekennzeichnet, dass** mindestens ein polymerer Verdicker in Form eines verzweigten oder vernetzten Polyelektrolyten enthalten ist, wobei der Polyelektrolyt ausgewählt ist aus einem Copolymer aus mindestens zwei Monomer-Typen, wobei mindestens ein Monomer eine starke Säurefunktion enthält, die teilweise oder vollständig neutralisiert ist, und mindestens ein Monomer
i) neutral ist oder
ii) eine schwache Säurefunktion enthäft.

14. Kosmetische Zusammensetzung gemäß einem der vorhergehenden Ansprüche, **dadurch gekennzeichnet, dass** die schwache Säurefunktion des Polyelektrolytmonomeren eine Carboxylgruppe -COOH darstellt, die teilweise oder vollständig neutralisiert ist.

15. Zusammensetzung gemäß einem der vorhergehenden Ansprüche, **dadurch gekennzeichnet, dass** die Monomere des Polyelektrolyten, die mit der schwach sauren -COOH-Gruppe funktionalisiert sind, ausgewählt sind aus Acrylsäure, Methacrylsäure, Itaconsäure und Maleinsäure, die teilweise oder vollständig neutralisiert sind.

16. Zusammensetzung gemäß einem der vorhergehenden Ansprüche, **dadurch gekennzeichnet, dass** die starke Säurefunktion des Polyelektrolytmonomeren ausgewählt ist aus einer Sulfonsäuregruppe -SO₃H und einer Phosphonsäuregruppe, die teilweise oder vollständig neutralisiert sind.

17. Zusammensetzung gemäß einem der vorhergehenden Ansprüche, **dadurch gekennzeichnet, dass** das Polyelektrolytmonomer mit starker Säurefunktion ausgewählt ist aus 2-Methyl-2[(1-oxo-2-propenyl)amino]-1-propansulfonsäure, die teilweise oder vollständig neutralisiert ist.

18. Zusammensetzung gemäß einem der vorhergehenden Ansprüche, **dadurch gekennzeichnet, dass** die Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Salz vorliegen.

19. Zusammensetzung gemäß einem der vorhergehenden Ansprüche, **dadurch gekennzeichnet, dass** das neutrale Polyelektrolytmonomer ausgewählt ist aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der genannten Ester mit einem Molekulargewicht zwischen 400 und 1000 g/mol, Acrylamid sowie Vinylpyrrolidon.

20. Zusammensetzung gemäß einem der vorhergehenden Ansprüche, **dadurch gekennzeichnet, dass** der Polyelektrolyt in Form eines inversen Polymerlatex, enthaltend eine Ölphase, eine Wasserphase und mindestens einen Öl-in-Wasser-Emulgator, enthalten ist.

21. Verwendung einer kosmetischen Zusammensetzung gemäß einem der Ansprüche 1 - 20 zur nicht-therapeutischen, kosmetischen Behandlung und/oder Minimierung von Hautfaften und -fältchen und/oder zur Verbesserung des optischen Erscheinungsbildes der Haut.

22. Nicht-therapeutisches, kosmetisches Verfahren zur nicht-therapeutischen, kosmetischen Behandlung und/oder Minimierung von Hautfalten und -fältchen und/oder zur Verbesserung des optischen Erscheinungsbildes der Haut, **dadurch gekennzeichnet, dass** eine kosmetische Zusammensetzung gemäß einem der Patentansprüche 1 - 20 auf die Haut aufgetragen wird.

## Claims

1. A cosmetic composition containing in a suitable cosmetic carrier
a) at least one silicone elastomer which is used in the form of an aqueous dispersion or is dispersed in the aqueous phase,
b) at least one silicone elastomer which is present in a silicone-based gel,
c) at least one aqueous phase,
d) at least one oil phase,
wherein the weight ratio of silicone elastomer a) to silicone elastomer b) is greater than 1 and wherein at least one particulate active ingredient, selected from organic cosmetic adsorbents with average particle diameters of 5-30 µm, preferably of 9-25 µm, which contains more than 50 wt.% spherical particles, is present in a total quantity of 3-10 wt.%, relative to the total weight of the composition.

2. A composition according to claim 1, **characterised in that** the weight ratio of silicone elastomer a) to silicone elastomer b) is in the range from 1.5-50, preferably 2-30 and particularly preferably 5-25.

3. A composition according to either one of the preceding claims, **characterised in that** at least one of the silicone elastomers a) or b) is obtainable by crosslinking an organopolysiloxane which contains in each molecule at least 2 C₂-C₁₀ alkenyl groups with a terminal double bond with an organopolysiloxane which comprises at least 2 silicone-bound hydrogen atoms in each molecule.

4. A composition according to any one of the preceding claims, **characterised in that** at least one of the silicone elastomers a) or b) is obtainable by crosslinking an organopolysiloxane which contains in each molecule at least 2 C₂-C₁₀ alkenyl groups with a terminal double bond with at least one alpha,omega-diene.

5. A composition according to any one of the preceding claims, **characterised in that** the silicone elastomer is selected from non-emulsifying and emulsifying silicone elastomers.

6. A composition according to any one of the preceding claims, **characterised in that** the organopolysiloxane with at least 2 C₂-C₁₀ alkenyl groups with a terminal double bond per molecule is selected from methylvinylsiloxanes, methylvinylsiloxane-dimethylsiloxane copolymers, dimethylpolysiloxanes with dimethylvinylsiloxy end groups, dimethylsiloxane-methylphenylsiloxane copolymers with dimethylvinyl-siloxy end groups, dimethylsiloxane-diphenylsiloxane-methylvinylsiloxane copolymers with dimethylvinylsiloxy end groups, dimethylsiloxane-methylvinylsiloxane copolymers with trimethylsiloxy end groups, dimethylsiloxane-methylphenylsiloxane-methylvinylsiloxane copolymers with trimethylsiloxy end groups, methyl-(3,3,3-trifluoropropyl)-polysiloxanes with dimethylvinylsiloxy end groups and dimethylsiloxane-methyl-(3,3,3-trifluoropropyl)-siloxane copolymers with dimethylvinylsiloxy end groups.

7. A composition according to any one of the preceding claims, **characterised in that** the crosslinking organopolysiloxane with at least two silicone bound hydrogen atoms is selected from methylhydrogenpolysiloxanes with trimethylsiloxy end groups, dimethylsiloxane-methylhydrogensiloxane copolymers with trimethylsiloxy end groups and cyclic dimethylsiloxane-methylhydrogensiloxane copolymers.

8. A composition according to any one of the preceding claims, **characterised in that** the at least one silicone elastomer a), which is present in dispersed form in the aqueous phase, is present in a total quantity of 0.5-30 wt.%, preferably 2.0-20 wt.% and particularly preferably 10-15 wt.%, in each case relative to the entire cosmetic composition.

9. A composition according to any one of the preceding claims, **characterised in that** the at least one silicone elastomer b), which is present in a silicone-based gel, is present in a total quantity of 0.05-5 wt.%, preferably 0.2-2 wt.% and particularly preferably 0.5-1 wt.%, in each case relative to the entire cosmetic composition.

10. A composition according to any one of the preceding claims, **characterised in that** the particulate active ingredient is selected from crosslinked polymethacrylates and crosslinked polymethyl methacrylates.

11. A composition according to any one of the preceding claims, **characterised in that** the particulate active ingredient comprises cavities.

12. A composition according to any one of the preceding claims, **characterised in that** the composition assumes the form of an oil-in-water emulsion.

13. A composition according to any one of the preceding claims, **characterised in that** at least one polymeric thickener is present in the form of a branched or crosslinked polyelectrolyte, wherein the polyelectrolyte is selected from a copolymer of at least two types of monomer, wherein at least one monomer contains a strong acid function which is partially or completely neutralised and at least one monomer
i) is neutral or
ii) contains a weak acid function.

14. A cosmetic composition according to any one of the preceding claims, **characterised in that** the weak acid function of the polyelectrolyte monomer is a carboxyl group -COOH which is partially or completely neutralised.

15. A composition according to any one of the preceding claims, **characterised in that** the polyelectrolyte monomers which are functionalised with the weakly acidic -COOH group are selected from acrylic acid, methacrylic acid, itaconic acid and maleic acid which are partially or completely neutralised.

16. A composition according to any one of the preceding claims, **characterised in that** the strong acid function of the polyelectrolyte monomer is selected from a sulfonic acid group -SO₃H and a phosphonic acid group which are partially or completely neutralised.

17. A composition according to any one of the preceding claims, **characterised in that** the polyelectrolyte monomer with a strong acid function is selected from 2-methyl-2[(1-oxo-2-propenyl)amino]-1-propanesulfonic acid which is partially or completely neutralised.

18. A composition according to any one of the preceding claims, **characterised in that** the polyelectrolyte monomers which are functionalised with a weak or strong acid group are present in partially or completely neutralised form as a sodium, potassium, ammonium, ethanolamine or amino acid salt.

19. A composition according to any one of the preceding claims, **characterised in that** the neutral polyelectrolyte monomer is selected from 2-hydroxyethyl acrylate, 2,3-dihydroxypropyl acrylate, 2-hydroxyethyl méthacrylate, 2,3-dihydroxypropyl methacrylate, the ethoxylated derivatives of the stated esters with a molecular weight of between 400 and 1000 g/mol, acrylamide as well as vinylpyrrolidone.

20. A composition according to any one of the preceding claims, **characterised in that** the polyelectrolyte is present in the form of an inverse polymer latex containing an oil phase, an aqueous phase and at least one oil-in-water emulsifier.

21. Use of a cosmetic composition according to any one of claims 1-20 for non-therapeutic, cosmetic treatment and/or for minimising skin folds and wrinkles and/or for improving the visual appearance of skin.

22. A non-therapeutic, cosmetic method for non-therapeutic, cosmetic treatment and/or for minimising skin folds and wrinkles and/or for improving the visual appearance of skin, **characterised in that** a cosmetic composition according to any one of claims 1-20 is applied onto the skin.

## Revendications

1. Composition cosmétique, contenant, dans un support cosmétique approprié,
a) au moins un élastomère de silicone, qui est utilisé sous forme d'une dispersion aqueuse ou qui est dispersé dans la phase aqueuse,
b) au moins un élastomère de silicone, qui se trouve dans un gel à base de silicone,
c) au moins une phase aqueuse,
d) au moins une phase huileuse,
le rapport pondéral d'élastomère de silicone a) à l'élastomère de silicone b) étant supérieur à 1 et au moins une substance active sous forme de particules, choisie parmi les adsorbants cosmétiques organiques, présentant des diamètres de particules moyens de 5-30 µm, de préférence de 9-25 µm, contenant des particules sphériques à raison de plus de 50% en poids, étant contenue en une quantité totale de 3-10% en poids, par rapport au poids total de la composition.

2. Composition selon la revendication 1, **caractérisée en ce que** le rapport pondéral d'élastomère de silicone a) à l'élastomère de silicone b) se situe dans la plage de 1,5-50, de préférence de 2-30 et de manière particulièrement préférée de 5-25.

3. Composition selon l'une quelconque des revendications précédentes, **caractérisée en ce qu'**au moins un des élastomères de silicone a) ou b) peut être obtenu par réticulation d'un organopolysiloxane, qui contient au moins 2 groupes C₂-C₁₀-alcényle avec une double liaison terminale dans chaque molécule, avec un organopolysiloxane qui présente au moins 2 atomes d'hydrogène liés par silicone dans chaque molécule.

4. Composition selon l'une quelconque des revendications précédentes, **caractérisée en ce qu'**au moins un des élastomères de silicone a) ou b) peut être obtenu par réticulation d'un organopolysiloxane, qui contient au moins 2 groupes C₂-C₁₀-alcényle avec une double liaison terminale dans chaque molécule, avec au moins un alpha-oméga-diène.

5. Composition selon l'une quelconque des revendications précédentes, **caractérisée en ce que** l'élastomère de silicone est choisi parmi les élastomères de silicone non émulsifiants et émulsifiants.

6. Composition selon l'une quelconque des revendications précédentes, **caractérisée en ce que** l'organopolysiloxane présentant au moins 2 groupes C₂-C₁₀-alcényle avec une double liaison terminale dans la molécule est choisi parmi les méthylvinylsiloxanes, les copolymères de méthylvinylsiloxane-diméthylsiloxane, les diméthylpolysiloxanes avec des groupes terminaux diméthylvinylsiloxy, les copolymères de diméthylsiloxane-méthylphénylsiloxane avec des groupes terminaux diméthylvinylsiloxy, les copolymères de diméthylsiloxane-diphénylsiloxane-méthylvinylsiloxane avec des groupes terminaux diméthylvinylsiloxy, les copolymères de diméthylsiloxane-méthylvinylsiloxane avec des groupes terminaux triméthylsiloxy, les copolymères de diméthylsiloxane-méthylphénylsiloxane-méthylvinylsiloxane avec des groupes terminaux triméthylsiloxy, les méthyl-(3,3,3-trifluoropropyl)-polysiloxanes avec des groupes terminaux diméthylvinylsiloxy et les copolymères de diméthylsiloxane-méthyl-(3,3,3-trifluoropropyl)-siloxane avec des groupes terminaux diméthylvinylsiloxy.

7. Composition selon l'une quelconque des revendications précédentes, **caractérisée en ce que** l'organopolysiloxane réticulant présentant au moins deux atomes d'hydrogène liés par silicone est choisi parmi les méthylhydrogénopolysiloxanes avec des groupes terminaux triméthylsiloxy, les copolymères de diméthylsiloxane-méthylhydrogénasiloxane avec des groupes terminaux triméthylsiloxy et les copolymères cycliques de diméthylsiloxane-méthylhydrogénosiloxane.

8. Composition selon l'une quelconque des revendications précédentes, **caractérisée en ce que** le au moins un élastomère de silicone a), qui se trouve sous forme dispersée dans la phase aqueuse, est contenu en une quantité totale de 0,5-30% en poids, de préférence de 2,0-20% en poids et de manière particulièrement préférée de 10-15% en poids, à chaque fois par rapport à la composition cosmétique totale.

9. Composition selon l'une quelconque des revendications précédentes, **caractérisée en ce que** le au moins un élastomère de silicone b), qui se trouve dans un gel à base de silicone, est contenu en une quantité totale de 0,05-5% en poids, de préférence de 0,2-2% en poids et de manière particulièrement préférée de 0,5-1% en poids, à chaque fois par rapport à la composition cosmétique totale.

10. Composition selon l'une quelconque des revendications précédentes, **caractérisée en ce que** la substance active sous forme de particules est choisie parmi les polyméthacrylates réticulés et les poly(méthacrylates de méthyle) réticulés.

11. Composition selon l'une quelconque des revendications précédentes, **caractérisée en ce que** la substance active sous forme de particules présente des espaces creux.

12. Composition selon l'une quelconque des revendications précédentes, **caractérisée en ce que** la composition se trouve sous forme d'émulsion huile-dans-eau.

13. Composition selon l'une quelconque des revendications précédentes, **caractérisée en ce qu'**au moins un épaississant polymère est contenu sous forme d'un polyélectrolyte ramifié ou réticulé, le polyélectrolyte étant choisi parmi un copolymère d'au moins deux types de monomères, au moins un monomère contenant une fonction d'acide fort, qui est partiellement ou totalement neutralisée, et au moins un monomère
i) étant neutre ou
ii) contenant une fonction d'acide faible.

14. Composition cosmétique selon l'une quelconque des revendications précédentes, **caractérisée en ce que** la fonction d'acide faible du monomère polyélectrolytique est un groupe carboxyle -COOH, qui est partiellement ou complètement neutralisé.

15. Composition selon l'une quelconque des revendications précédentes, **caractérisée en ce que** les monomères du polyélectrolyte, qui sont fonctionnalisés par le groupe acide faible -COOH, sont choisis parmi l'acide acrylique, l'acide méthacrylique, l'acide itaconique et l'acide maléique, qui sont partiellement ou complètement neutralisés.

16. Composition selon l'une quelconque des revendications précédentes, **caractérisée en ce que** la fonction d'acide fort du monomère polyélactrolytique est choisie parmi un groupe acide sulfonique -SO₃H et un groupe acide phosphonique, qui sont partiellement ou complètement neutralisés.

17. Composition selon l'une quelconque des revendications précédentes, **caractérisée en ce que** le monomère polyélectrolytique présentant une fonction d'acide fort est choisi parmi l'acide 2-méthyl-2-[(1-oxo-2-propényl)amino]-1-propanesulfonique, qui est partiellement ou complètement neutralisé.

18. Composition selon l'une quelconque des revendications précédentes, **caractérisée en ce que** les monomères polyélectrolytiques, qui sont fonctionnalisés par un groupe acide faible ou fort, se trouvent sous forme partiellement ou complètement neutralisée sous forme de sel de sodium, de potassium, d'ammonium, d'éthanolamine ou d'acide aminé.

19. Composition selon l'une quelconque des revendications précédentes, **caractérisée en ce que** le monomère polyélectrolytique neutre est choisi parmi l'acrylate de 2-hydroxyéthyle, l'acrylate de 2,3-dihydroxypropyle, le méthacrylate de 2-hydroxyéthyle, le méthacrylate de 2,3-dihydroxypropyle, les dérivés éthoxylés des esters mentionnés présentant un poids moléculaire entre 400 et 1000 g/mole, l'acrylamide ainsi que la vinylpyrrolidone.

20. Composition selon l'une quelconque des revendications précédentes, **caractérisée en ce que** le polyélectrolyte est contenu sous forme d'un latex polymère inversé, contenant une phase huileuse, une phase aqueuse et au moins un émulsifiant huile-dans-eau.

21. Utilisation d'une composition cosmétique selon l'une quelconque des revendications 1-20 pour le traitement non thérapeutique, cosmétique et/ou la minimisation de rides et de ridules de la peau et/ou pour améliorer l'aspect visuel de la peau.

22. Procédé non thérapeutique, cosmétique pour le traitement non thérapeutique, cosmétique et/ou pour la minimisation de rides et de ridules de la peau et/ou pour améliorer l'aspect visuel de la peau, **caractérisé en ce qu'**une composition cosmétique selon l'une quelconque des revendications 1-20 est appliquée sur la peau.
